(19) Europäisches Patentamt
European Patent Office
Office européen des brevets

(11) **EP 3 813 895 B1**

(12) **EUROPEAN PATENT SPECIFICATION**

(45) Date of publication and mention
of the grant of the patent:
**30.07.2025 Bulletin 2025/31**

(21) Application number: **19733058.2**

(22) Date of filing: **28.06.2019**

(51) International Patent Classification (IPC):
**A61L 27/20** *(2006.01)*    **A61L 27/52** *(2006.01)*
**A61L 27/54** *(2006.01)*

(52) Cooperative Patent Classification (CPC):
(C-Sets available)
**A61L 27/52; A61L 27/20; A61L 27/54;**
A61L 2300/402; A61L 2300/622; A61L 2400/06;
A61L 2430/34                              (Cont.)

(86) International application number:
**PCT/EP2019/067322**

(87) International publication number:
**WO 2020/002597 (02.01.2020 Gazette 2020/01)**

(54) **FATTY ACID-GRAFTED HYALURONIC ACID, DERMAL FILLER FORMULATIONS COMPRISING SAME, PROCESS FOR PREPARATION AND USE THEREOF**

FETTSÄUREGEPFROPFTE HYALURONSÄURE, DERMALE FÜLLSTOFFFORMULIERUNGEN DAMIT, VERFAHREN ZUR HERSTELLUNG UND VERWENDUNG DAVON

ACIDE HYALURONIQUE GREFFÉ SUR DE L'ACIDE GRAS, FORMULATIONS DE REMPLISSAGE DERMIQUE LE COMPRENANT , PROCÉDÉ DE PRÉPARATION ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.06.2018 EP 18180909**

(43) Date of publication of application:
**05.05.2021 Bulletin 2021/18**

(73) Proprietor: **Merz Pharma GmbH & Co. KGaA 60318 Frankfurt am Main (DE)**

(72) Inventors:
 • **HÖNNSCHEIDT, Christoph
   60438 Frankfurt am Main (DE)**
 • **KÖHLER, Holger
   55218 Ingelheim (DE)**

(74) Representative: **Fritsche, Erich Roland
   Wallinger Ricker Schlotter Tostmann
   Patent- und Rechtsanwälte Partnerschaft mbB
   Zweibrückenstraße 5-7
   80331 München (DE)**

(56) References cited:
 WO-A1-2014/082609        WO-A1-2016/074794
 WO-A1-2018/083195        US-A1- 2011 224 164
 US-A1- 2015 151 005

 • HUERTA-ANGELES GLORIA ET AL: "Novel synthetic method for the preparation of amphiphilic hyaluronan by means of aliphatic aromatic anhydrides", CARBOHYDRATE POLYMERS, APPLIED SCIENCE PUBLISHERS, LTD. BARKING, GB, vol. 111, 27 May 2014 (2014-05-27), pages 883 - 891, XP029035908, ISSN: 0144-8617, DOI: 10.1016/ J.CARBPOL.2014.05.035
 • HUERTA-ANGELES GLORIA ET AL: "Linolenic acid grafted hyaluronan: Process development, structural characterization, biological assessing, and stability studies", CARBOHYDRATE POLYMERS, APPLIED SCIENCE PUBLISHERS, LTD. BARKING, GB, vol. 152, 9 July 2016 (2016-07-09), pages 815 - 824, XP029679039, ISSN: 0144-8617, DOI: 10.1016/ J.CARBPOL.2016.07.030

EP 3 813 895 B1

(52) Cooperative Patent Classification (CPC): (Cont.)

    C-Sets
    **A61L 27/20, C08L 5/08**

**Description**

## FIELD OF THE INVENTION

The present invention relates to a sterile microsphere-based dermal filler formulation and a sterile crosslinked hyaluronic acid-based dermal filler formulation each comprising a fatty acid-grafted hyaluronic acid (FA-g-HA). The present invention further relates to processes for the preparation of the FAg-HA and the dermal filler formulations comprising the FA-g-HA, and their use in cosmetic applications.

## BACKGROUND OF THE INVENTION

Skin ageing is a natural degenerative process of the skin caused by intrinsic (chronological) and extrinsic (environmental) mechanisms. The main signs of skin ageing are loss of volume and reduced skin elasticity, which manifest themselves for example by wrinkles, lax and thinner skin, sagging, cheek cavities and protrusion of facial musculature. These skin changes are largely due to age-related progressive loss and fragmentation of collagen fibrils in the dermal extracellular matrix (ECM).

In recent studies, it was uncovered that deflation and volume changes of fat compartments of the face and other body parts represent another cause of skin ageing. In particular, the deflation and loss of the normal anatomic subcutaneous facial fat compartments gives off the appearance of increased skin laxity or prominent folds around the nasolabial region, periorbital region, and jowl (see, e.g., Farkas et al., Plast. Reconstr. Surg. - Global Open 2013; 1:e8).

A wide range of anti-aging treatments have been developed over the past decades that aim at rejuvenating the skin to restore a youthful appearance. In recent years, major facial operations like "face-lifting" have been increasingly replaced by non-surgical procedures. For example, the above-mentioned deflation and loss of fat compartments has been treated by focused localized fat injection into discrete compartments, e.g. the deep medial and middle fat pads of the cheek. Furthermore, the injection of soft tissue augmentation products (commonly called "fillers") has become particularly popular. Among the various filler products, dermal fillers based on hyaluronic acid (HA) have been increasingly used in the last years and are now considered the "gold standard" for restoring volume to the skin.

Hyaluronic acid is a naturally occurring linear polysaccharide consisting of alternating $\beta$-1,4-linked units of $\beta$-1,3-linked glucuronic acid and N-acetyl-D-glucosamine. It is well-known for its excellent biocompatibility, non-immunogenicity, safety, water-binding capacity, and rheological properties. HA-based fillers are used in a broad range of applications including, for example, removal of fine lines or wrinkles, plumbing of lips, sculpting of cheeks, altering the face contours (e.g., of the nose or chin), soften facial creases, and improving the appearance of recessed scars.

If injected in its naturally occurring form, HA rapidly degrades *in vivo* mainly through enzymatic hydrolysis by hyaluronidase. Therefore, HA is commonly "stabilized" by chemical crosslinking using a crosslinking agent such as the industry standard crosslinker 1,4-butanediol diglycidyl ether (BDDE), resulting in a prolonged *in vivo* persistence (> 6 months). The type and degree of crosslinking is a major determinant of the viscoelastic and chemical properties of fillers, such as lifting capacity, ease of injection and *in vivo* persistence.

Besides covalent crosslinking, a variety of other chemically modified HAs are known in the art such as acylated, amidated, esterified and O-sulfonated HA derivatives, including conjugates of HA with peptides, proteins, vitamins, and fatty acids. These modifications impart specific physicochemical characteristics to HA that can be specifically tailored to the desired application. Such modified HA are frequently reported in the literature to be capable of forming polymer-drug conjugates for drug delivery and spherical aggregates for encapsulation and delivery of drugs.

In this regard, Smejkalova et al. (Carbohydrate Polymers 87 (2012), 1460-1466) disclose acylation of a starting NaHA material ($M_w$ = 50 kDa) with a symmetric hexanoic anhydride in a DMSO/$H_2O$ mixed solvent in the presence of dimethylaminopyridine (DMAP) and triethylamine (TEA). The resulting C6-acyl (i.e. hexanoic acid)-HA derivative was found to have a degree of substitution (DS) of 70% and is supposed to be suitable for use as drug delivery system.

In addition, Huerta-Angeles et al. (Carbohydrate Polymers 111 (2014), 883-891) describe a method of synthesis of amphiphilic hyaluronan (HA) by esterification with fatty acids using a mixed aliphatic aromatic anhydride of general formula $RCOOCOC_6H_2Cl_3$ prepared by reaction of fatty acids (R) with 2,4,6-trichlorobenzoyl chloride (TCBC) in the presence of trimethylamine (TEA) and tetrahydrofuran (THF). The aliphatic aromatic anhydride was added to a solution of HA in $H_2O$ and dimethylamine pyridine (DMAP) to afford fatty acid-modified HA. However, the maximum degree of substitution (DS) achievable using a low molecular weight HA starting material ($M_w$ = 15 kDa) was found to decrease with increasing length of acyl chain. In fact, TCBC failed to activate long-chain fatty acids ($C_{20}$ and longer), as indicated by a DS of 0%. Also, increasing the $M_w$ of HA to 15 kDa was found to lower the DS (using cis-oleic acid (C18:1) as fatty acid) to below 10%. Moreover, Huerta-Angeles et al. report that sodium oleyl hyaluronate (DS = 12%; $M_w$ = 15 kDa) is capable of forming vehicles having an average diameter size of about 60 nm, which are potentially suitable for encapsulation of non-polar drugs.

WO 2014/082609 discloses the preparation of fatty acid derivatives of HA by esterification using a halogenide derivative of 2,4,6-trichlorobenzoic acid (e.g. TCBC) for activating the fatty acids. Reportedly, the hydrophobized HA can be used for preparation of nanomicelles of 20 nm to 100 nm comprising hydrophobized HA and a biologically active substance encapsulated therein. These HA nanomicelles are disclosed to support the penetration of bound substances in topical applications and to be usable in cosmetic and pharmaceutical applications. US2011/224164 discloses dermal filler compositions comprising an uncrosslinked hyaluronan having high and low molecular weight hyaluronan, wherein the molecular weight is from 1.000.000 Da to 5.000.000 Da.

A number of polymers other than HA have also been investigated with regard to their capacity of forming nanoparticulate systems, but for use as drug delivery systems. For example, a polyvinyl alcohol (PVA)-based nanoformulation with encapsulated ubiquinone was described in Hoennscheidt et al. (Int. J. Pharm. 478 (2015), 416-425). It was also reported that the modification of high molecular $\gamma$-polyglutamic acid with hydrophobic L-phenylalanine ethyl ester gives an amphiphilic comb polymer capable of encapsulating the poorly water-soluble drug quinine (Hoennscheidt et al., Biochem. Eng. 79 (2013), 259-266; Christoph Hoennscheidt, thesis entitled "Entwicklung kolloiddisperser Wirkstoffformulierungen auf Basis von Biopolymeren", 2016, Cuvillier, Goettingen,176 pages).

Despite the fact that a variety of different injectable dermal fillers have been developed in the past years covering a broad range of applications, the demand for dermal fillers is continuously growing and consumer's expectations are rising. Recent advances resulted in smoother results and higher rates of patient satisfaction. However, there still remain concerns regarding safety, adverse reactions, allergic reactions, durability, physical and mechanical properties, ease of use, and pain upon injection.

For these reasons, great efforts have been directed to the optimization of viscoelastic properties, which are essential to dermal filler product performance. Whilst increasing the concentration of HA and/or the crosslinking degree generally leads to an enhanced volumizing effect and prolonged persistence this, however, results in an increased viscosity of the HA-based dermal filler. As a result, the force required to extrude the filler through thin needles is undesirably increased for the practitioner or - if thicker needles are used - the patient's pain and discomfort during injection is undesirably augmented.

Thus, an optimal dermal filler material should meet several requirements and, in particular, provide an optimal balance of persistence, lifting capacity and ease of injection.

## OBJECT OF THE INVENTION

In view of the above, the object of the present invention is to provide a safe hyaluronic acid (HA)-based dermal filler formulation that has a sufficiently high lifting capacity and longevity while at the same time being easily injectable through fine needles.

## SUMMARY OF THE INVENTION

The present invention and the scope therof is defined by the appended claims. The more generic description of the invention is provided for illustrative purposes only. Embodiments not falling under these claims are for reference purpose only. According to the present invention, the above object is solved by a sterile microsphere-based dermal formulation and a sterile crosslinked hyaluronic acid-based dermal filler formulation, comprising a fatty acid-grafted hyaluronic acid (FA-g-HA), the HA of the FA-g-HA having a hyaluronic acid backbone of 1,250 to 12,500 repeating disaccharide units and the fatty acid having 6 to 22 carbon atoms, preferably having 8 to 18 carbon atoms.

The FA-g-HA was found to spontaneously form microspheres in aqueous solution by self-aggregation, resulting in a formulation of dispersed microspheres of FA-g-HA that is suitable for use as dermal filler. This microsphere-based dermal filler formulation was found to have an advantageously low viscosity to facilitate injection while still having a sufficiently high stability and lifting capacity for use as a dermal filler in various cosmetic (aesthetic) applications, making it a promising candidate of a HA material for the next generation of "highly liquid" HA filler.

In addition, it was unexpectedly found that the FA-g-HA is excellent for use as a lubricant (or "lubrication phase") in HA-based dermal fillers, i.e. it is capable of decreasing the injection force required to extrude a HA-based dermal filler through a needle of a syringe. Further advantages of the dermal filler formulations of the present invention and additional benefits, such as nutritional effects on cells, are described further below.

In a first aspect, not falling within the scope of the claimed subject-matter, the present invention provides a fatty acid-grafted hyaluronic acid having a hyaluronic backbone of n repeating disaccharide units with n ranging from 1,250 to 12,500, in particular from 2,500 to 10,000, wherein the fatty acid is a C6-C22 fatty acid.

In a second aspect, the present invention provides a process for the preparation of a fatty acid-grafted hyaluronic acid having a hyaluronic acid backbone of 1,250 to 12,500 repeating disaccharide units, wherein the fatty acid is a C6-C22 fatty acid, comprising the steps of:

(a) dispersing hyaluronic acid in an organic solvent, in particular a non-polar solvent, to obtain an organic dispersion,

(b) adding fatty acid chlorides to the organic dispersion,

(c) allowing the fatty acid chlorides to esterify with hyaluronic acid under reaction conditions sufficient to form a fatty acid-grafted hyaluronic acid, and

(d) isolating the fatty acid-grafted hyaluronic acid.

In a third aspect, the present invention relates to the use of the fatty acid-grafted hyaluronic acid according to claims 2 and 3 in the preparation of a dermal filler formulation, in particular to the use as a lubricant in a dermal filler formulation.

In a fourth aspect, the present invention provides a sterile microsphere-based dermal filler formulation, comprising microspheres of fatty acid-grafted hyaluronic acid according to claims 4 and 5.

In a fifth aspect, the present invention relates to a process for the preparation of a sterile microsphere-based dermal filler formulation according to the fourth aspect of the present invention, comprising the steps of:

(A) contacting a fatty acid-grafted hyaluronic acid having a hyaluronic acid backbone of 1,250 to 12,500 repeating disaccharide units, wherein the fatty acid is a C6-C22 fatty acid, with an aqueous solution to obtain a microsphere-based formulation of fatty acid-grafted hyaluronic acid,

(B) optionally subjecting the microsphere-based formulation of fatty acid-grafted hyaluronic acid to a high pressure homogenization (HPH) treatment, and

(C) sterilizing the optionally HPH-treated, microsphere-based formulation to obtain a sterile microsphere-based dermal filler formulation.

In a sixth aspect, the present invention provides a sterile crosslinked hyaluronic acid-based dermal filler formulation, comprising hyaluronic acid crosslinked with a crosslinking agent, in particular 1,4-butanediol diglycidyl ether (BDDE), and a fatty acid-grafted hyaluronic acid according to claim 7.

In a seventh aspect, the present invention provides a process for the preparation of a sterile crosslinked hyaluronic acid-based dermal filler formulation according to the sixth aspect of the present invention, comprising the steps of:

(i) providing a fatty acid-grafted hyaluronic acid having a hyaluronic acid backbone of 1,250 to 12,500 repeating disaccharide units, wherein the fatty acid is a C6-C22 fatty acid,

(ii) providing a crosslinked hyaluronic acid,

(iii) combining the fatty acid-grafted hyaluronic acid and the crosslinked hyaluronic acid to obtain a crosslinked hyaluronic acid-based dermal filler formulation, and

(iv) sterilizing the crosslinked hyaluronic acid-based dermal filler formulation to obtain a sterile crosslinked hyaluronic acid-based dermal filler formulation.

In an eighth aspect, the present invention provides a prefilled syringe, comprising a sterile dermal filler composition according to the fourth or sixth aspect of the present invention.

In a ninth aspect, the present invention relates to the use of a sterile dermal filler formulation according to the fourth or sixth aspect of the present invention for cosmetic treatments, in particular for augmenting or filling of wrinkles or fine lines of the skin (e.g., nasolabial folds, marionette lines, chin folds, lower jawlines, oral commissure, and the like), filling cutaneous depressions, masking scars, increasing the volume of the lips, augmenting cheeks, nose corrections, and/or improve skin hydration and skin texture.

Also disclosed, but not part of the claimed invention, is a method for augmenting or filling soft tissues, comprising administering to a subject in need thereof an effective amount of a sterile dermal filler formulation according to the fourth or sixth aspect of the present invention.

## DETAILED DESCRIPTION OF THE INVENTION

The present invention is based on the observation that the covalent incorporation of lipophilic fatty acid groups in high molecular weight hyaluronic acid (HA) leads, in an aqueous environment, to the formation of microspheres having an amphiphilic character, i.e. a hydrophilic microsphere surface and a lipophilic microsphere core.

Specifically, it was unexpectedly found that dermal filler compositions comprising and based on said microspheres of fatty acid-grafted hyaluronic acid (FAg-HA) exhibit improved fluid dynamics, offering a range of tentative advantages compared to conventional HA dermal fillers.

One significant advantage of the microsphere-based dermal filler formulation of the present invention is its low viscosity and therefore reduced injection force, facilitating the injection of the formulation into the target tissue. Furthermore, the reduced viscosity allows one to use finer needles (e.g., 30G vs. 27G) than those used for conventional crosslinked HA-based fillers, which in turn results in an improved injection comfort for the patient. Furthermore, since significantly reduced injection force is needed, this leads to a significant reduction in severity of immediate injection-site discomfort and early injection side reactions (e.g., swelling, redness, and haematoma).

Due to the possibility of using very fine needles and reduced injection site irritation, the microsphere-based hydrogel formulation of the present invention has the potential to be injected without a local anesthetic agent such as lidocaine. This reduces the risk of drug-attributable side effects. In other words, it is no longer desired or required to premix or incorporate a local anesthetic agent into the dermal filler or to perform local anaesthesia prior to filler injection.

The microsphere-based hydrogel formulation of the present invention also offers beneficial rheological properties, including unique shaping and texturizing characteristics. At the same time, the stability of the formulation is much higher than that of native HA. In addition, no toxic crosslinker (e.g., BDDE) is used for preparing the microsphere-based hydrogel formulation of the present invention, resulting in an improved safety profile.

Without being bound by theory, it is believed that the high viscosity of HA in water is due to intermolecular ionic interactions of the polymer chains forming a stable network. The network formation involves transition from viscoelastic fluid properties to viscoelastic solid-state properties. The grafting of lipophilic groups to the macromolecular backbone of the HA is believed to disrupt this network formation. The extent of the disturbed network formation depends on the lipophilic substance fraction in the macromolecule. This allows the preparation of formulations with greatly reduced viscosity (more liquid formulations) and/or highly concentrated HA gel formulations with modified degradation kinetics.

Moreover, it was also found, in an unexpected way, that the FA-g-HA is excellent for use as a lubricant (or "lubrication phase") in HA-based dermal fillers, i.e. that it is capable of decreasing the injection force required to extrude a HA-based dermal filler through a needle of a syringe. Also, the FA-g-HA generally has a certain impact on the viscoelastic characteristics of HA-based dermal fillers and may therefore be used to fine-tune the desired mechanical and rheological filler properties. Furthermore, as compared to uncrosslinked HA that is commonly used as lubricant in HA-based fillers. FA-g-HA shows a decelerated degradation during UV exposure and an increased longevity. Without being bound by theory, it is believed that these beneficial properties are due to a protecting or "shielding" effect of the grafted fatty acids and to the formation of microspheres, thereby stabilizing the HA polymer against enzymatic and/or radical degradation.

The use of FA-g-HA as lubricant provides a number of additional benefits to dermal filler compositions including the ability of the hydrophobic fatty acids of the FA-g-HA to confer a nature to the dermal filler composition that is more similar to the surroundings at the site of injection, in particular if applied into subcutaneous tissue. Due to its more similar nature to the tissues surrounding the site of injection, undesired side-reactions like inflammation are abolished or diminished. Yet further beneficial effects are discussed herein below, for example in connection with the third aspect of the present invention.

According to a first aspect of the present invention, not falling within the scope of the claimed subject-matter, there is provided a fatty acid-grafted hyaluronic acid having a hyaluronic backbone of n repeating disaccharide units with n ranging from 1,250 to 12,500, in particular from 2,500 to 10,000, wherein the fatty acid is a C6-C22 fatty acid.

As used herein, the term "fatty acid-grafted hyaluronic acid" or "FA-g-HA" refers to a derivative of hyaluronic acid having fatty acids covalently attached to the hyaluronic acid backbone, in particular esterified with the hyaluronic acid backbone. More specifically, the fatty acids of FA-g-HA generally exist in ester linkage with hydroxyl groups of hyaluronic acid via their carboxyl group. Preferably, the fatty acids are esterified with a primary hydroxyl group of hyaluronic acid, i.e. with the primary hydroxyl group at position 6 of the N-acetyl-D-glucosamine monomer. However, this does mean that one or more of the secondary hydroxyl groups of a given repeating disaccharide unit of hyaluronic acid does not carry a fatty acid group covalently bound by an ester linkage.

The term "hyaluronic acid" or "HA", as used herein, includes hyaluronic acid, hyaluronate, and any hyaluronate salts such as sodium hyaluronate and mixtures of salts. Likewise the term "fatty acid-grafted hyaluronic acid" or "FA-g-HA" includes any protonated or deprotonated forms and any salts thereof.

In accordance with the present invention, hyaluronic acid backbone of the FA-g-HA comprises n repeating disaccharide units, wherein n is preferably from 1,250 to 12,500, preferably from 2,500 to 12,500, and more preferably from 3,750 to 10,000 or from 5,000 to 7,500. For the purpose of the present invention, the molecular mass of a repeating disaccharide unit of HA may be taken as 400 Da for calculating the corresponding molecular mass. Thus, alternatively, the hyaluronic acid backbone may be characterized by having an average molecular mass of from $0.5 \times 10^6$ Da to $5.0 \times 10^6$ Da, preferably from $1.0 \times 10^6$ Da to $5.0 \times 10^6$ Da, and more preferably from $1.5 \times 10^6$ Da to $4.0 \times 10^6$ Da or from $2.0 \times 10^6$ Da to $3.0 \times 10^6$ Da.

Within the framework of the present invention, the average molecular mass of HA polymers is preferably determined by viscosimetry via the Mark-Houwink equation. The Mark-Houwink equation gives a relation between intrinsic viscosity ($\eta$) and the viscosity average molecular weight and allows determination of the average molecular weight of a polymer from data on the intrinsic viscosity and vice versa. Within the context of the present invention, the intrinsic viscosity is preferably measured according to the procedure defined in European Pharmacopoeia 7.0 (Hyaluronic Acid monograph No. 1472, 01/2011). For calculation of the average molecular weight of HA from intrinsic viscosity data, the following Mark-Houwink is used within the framework of the present invention:

$$[\eta] = K \times M^a,$$

wherein $[\eta]$ = intrinsic viscosity in $m^3/kg$, M = viscosity average molecular weight, K = $2.26 \times 10^{-5}$, and a = 0.796. In accordance with the present invention, the intrinsic viscosity of the HA starting material usually ranges from $1.350 \ m^3/kg$ (1350 ml/g) to $4.500 \ m^3/kg$ (4500 ml/g), especially from $2.0 \ m^3/kg$ to $4.0 \ m^3/kg$.

Typically, the hyaluronic acid used for making the FA-g-HA is a single hyaluronic acid material. However, the hyaluronic acid starting material is not particularly limited and may, for example, include a mixture of two or more hyaluronic acid preparations having different average molecular weights, such as a first average molecular weight below $1.0 \times 10^6$ Da and a second average molecular weight of above $1.0 \times 10^6$ Da, wherein the difference between the first and second molecular weights is preferably more than $0.1 \times 10^6$ Da, more than $0.5 \times 10^6$ Da, or more than $1.0 \times 10^6$ Da.

Furthermore, the hyaluronic acid is not limited by a particular molecular weight distribution. The polydispersity index (PDI) is a measure of the distribution of molecular mass in a given polymer sample and is calculated according to the following formula:

$$PDI = M_w/M_n,$$

wherein Mw is the weight average molecular weight and Mn is the number average molecular weight. Mw and Mn can both be determined by gel permeation chromatography (GPC). Within the framework of the present invention, the PDI may be, e.g., from 1 to 1.5 or from 1.1 to 1.3.

The fatty acid grafted to the hyaluronic acid is typically a carboxylic acid with an aliphatic hydrocarbon chain having 6 to 22 carbon atoms, which is either saturated or unsaturated and preferably unbranched (linear). A saturated fatty acid has an aliphatic hydrocarbon chain with only single bonds between its carbon atoms, whereas an unsaturated fatty acid has an aliphatic hydrocarbon chain with at least one double bond or, more specifically, a carbon-carbon double bond.

The fatty acid is a C6-C22 fatty acid, preferably a C7-C20 fatty acid or a C6-C18 fatty acid, and more preferably a C7-C18 fatty acid, a C8-C16 fatty acid, a C9-C14 fatty, a C10-C12 fatty acid, and most preferably a C6-C10 or C6-C8 fatty acid, wherein these fatty acids are preferably (i) linear (i.e. unbranched) and saturated or (ii) linear and unsaturated (e.g., monounsaturated or polyunsaturated, preferably monounsaturated), or any mixture thereof. A particularly suitable fatty acid for use herein is a C8-C18, C8-C16, C10-C14 or C10-C12 fatty acid, which is linear and saturated (preferably monounsaturated) or unsaturated.

Specific examples of unsaturated fatty acids include:

caproic acid ($CH_3(CH_2)_4COOH$; C6:0),
caprylic acid ($CH_3(CH_2)_6COOH$; C8:0),
capric acid ($CH_3(CH_2)_8COOH$; C10:0),
lauric acid ($CH_3(CH_2)_{10}COOH$; C12:0),
myristic acid ($CH_3(CH_2)_{12}COOH$; C14:0),
palmitic acid ($CH_3(CH_2)_{14}COOH$; C16:0),
stearic acid ($CH_3(CH_2)_{16}COOH$; C18:0),
arachidic acid ($CH_3(CH_2)_{18}COOH$; C20:0),
behenic acid ($CH_3(CH_2)_{20}COOH$; C22:0), and
lignoceric acid ($CH_3(CH_2)_{22}COOH$; C24:0). Not falling within the scope of the claimed subject-matter.

The saturated fatty acid is preferably selected from the group consisting of caproic acid, caprylic acid, capric acid, lauric acid, myristic acid, palmitic acid, stearic acid, and mixtures thereof, more preferably from the group consisting of caprylic acid, capric acid, lauric acid, myristic acid, palmitic acid, and mixtures thereof, or from the group consisting of capric acid, lauric acid, myristic acid, and mixtures thereof.

Specific Examples of unsaturated fatty acids include:

myristoleic acid (*cis*-$CH_3(CH_2)_3CH=CH(CH_2)_7COOH$; C14:1 9-*cis*),
palmitoleic acid (*cis*-$CH_3(CH_2)_5CH=CH(CH_2)_7COOH$; C16:1 9-*cis*),
sapienic acid (*cis*-$CH_3(CH_2)_8CH=CH(CH_2)_4COOH$; C16:1 6-*cis*),
oleic acid (*cis*-$CH_3(CH_2)_7CH=CH(CH_2)_7COOH$; C18:1 9-*cis*),
elaidic acid (*trans*-$CH_3(CH_2)_7CH=CH(CH_2)_7COOH$; C18:1 9-*trans*),
vaccenic acid (*cis*-$CH_3(CH_2)_5CH=CH(CH_2)_9COOH$; C18:1 11-*cis*
linoleic acid (9,12-*cis*-$CH_3(CH_2)_4CH=CHCH_2CH=CH(CH_2)_7COOH$; C18:1 9,12-*cis*),
linoelaidic acid (9,12-*trans*-$CH_3(CH_2)_4CH=CHCH_2CH=CH(CH_2)_7COOH$; C18:1), and
erucic acid (*trans*-$CH3(CH2)7CH=CH(CH2)11COOH$; C22:1 13-*trans*).

The unsaturated fatty acid is preferably selected from the group consisting of myristoleic acid, palmitoleic acid, oleic acid, and linoleic acid, more preferably form the group consisting of myristoleic acid, palmitoleic acid, and oleic acid. Other suitable unsaturated fatty acids include C8, C10 and C12 fatty acids with one double bond.

The term "fatty acid-grafted hyaluronic acid" or "FA-g-HA", as used herein, is not intended to impose any particular limitation on the amount, number or degree of fatty acids grafted to the hyaluronic acid. However, the degree of fatty acid modification ($MoD_{FA}$), defined as the total number of grafted fatty acids to the total number of repeating disaccharide units of hyaluronic acid in percent, is usually at least 1%. This means that in average at least 1 disaccharide unit carries a grafted fatty acid per 100 disaccharide units. Typically, the degree of fatty acid modification ($DoM_{FA}$) is between 1% and 70% or between 1% and 60%. Preferably, the degree of fatty acid modification is 1% to 50% or 2% to 40%, more preferably 3% to 30% or 4% to 25% or 5% to 20%.

It is pointed out that, within the meaning of the present invention, more than one functional group, i.e. hydroxyl group, of a repeating disaccharide unit of hyaluronic acid may be esterified with a fatty acid. For example, it may be, and is within the scope of the present invention, that the primary hydroxyl group of a *N*-acetyl-D-glucosamine residue is grafted with a fatty acid via an ester bond and, in addition, one or more other secondary hydroxyl groups of the same repeating disaccharide unit is also grafted with a fatty acid.

The degree of fatty acid modification may be determined by proton nuclear magnetic resonance ($^1$H NMR) measurements, as known to those skilled in the art. For example, preparation is done by dissolving solid material in $D_2O$ and subsequent enzymatic breakdown of the HA backbone. As a result, there are characteristic NMR signals representing parts of the fatty acids as well as for the HA, which give a quantitative relation between both components. In particular, the methyl signal for the fatty acids (at approx. 1 ppm) and the methyl group of HA (at approx. 2 ppm) may be used for calculation the degree of modification. The NMR measurements as such may be performed as described herein below in relation to the sixth aspect of the present invention.

According to a second aspect of the present invention, there is provided a process for the preparation of a fatty acid-grafted hyaluronic acid having a hyaluronic acid backbone of 1,250 to 12,500 repeating disaccharide units, wherein the fatty acid is a C6-C22 fatty acid, comprising the steps of:

(a) dispersing hyaluronic acid in an organic solvent, in particular a non-polar solvent, to obtain an organic dispersion,
(b) adding fatty acid chlorides to the organic dispersion,
(c) allowing the fatty acid chlorides to esterify with hyaluronic acid under reaction conditions sufficient to form a fatty acid-grafted hyaluronic acid, and
(d) isolating the fatty acid-grafted hyaluronic acid.

In step (a), the organic solvent is preferably a non-polar solvent such as n-pentane, chloroform, tetrahydrofuran, heptane, toluene, benzene, and mixtures thereof. Particularly suitable for use herein is n-pentane.

The hyaluronic acid dispersed in step (a) is pretreated by dispersing native hyaluronic acid, e.g. in the form of fibers, in an aqueous solution. The obtained aqueous dispersion is then freeze-dried to produce a freeze-dried hyaluronic acid material. This material is then used in step (a) as hyaluronic acid, which is dispersed in an organic solvent to obtain an organic dispersion. It was surprisingly found that the pre-treatment results in an improved reaction of the fatty acid chlorides with hyaluronic acid. Without being bound by theory, it is believed that this is due to an increased and/or altered surface of the hyaluronic acid pre-treated this way.

In step (b), the one or more fatty acid chlorides are compounds of formula R-C(=O)Cl, wherein R is an aliphatic hydrocarbon chain having 5 to 21 carbon atoms, and the aliphatic hydrocarbon chain is either saturated or unsaturated and preferably unbranched (linear). This is, the fatty acid chlorides suitable for use herein correspond to the fatty acids described herein above, with the same preference, except that a chlorine atom is bonded to the carbonyl carbon atom instead of a hydroxyl group.

Step (c) may be carried out at different reaction conditions (e.g., temperature and reaction time) as long as this step results in the production of fatty acid-grafted hyaluronic acid. For example, step (c) may be carried out at room temperature (e.g., 20-25°C) and for about 12 hours (e.g., 8 to 16 hours).

In step (d), the reaction product obtained from step (c) is purified to terminate the grafting reaction and separate unbound fatty acids and reaction side products. Preferably, step (d) includes sub-step (d1) of separating the fatty acid-grafted hyaluronic acid and dissolving it in an aqueous solution to obtain an aqueous dispersion of fatty acid-grafted hyaluronic acid and sub-step (d2) of freeze-drying the aqueous dispersion of fatty acid-grafted hyaluronic acid to obtain a freeze-dried fatty acid-grafted hyaluronic acid.

According to a third aspect, the present invention relates to the use of the fatty acid-grafted hyaluronic acid according to claims 2 and 3 in the preparation of a dermal filler formulation, in particular to the use as a lubricant in a dermal filler formulation.

The term "dermal filler" or "dermal filler formulation", as used herein, is intended to refer to a formulation designed to add volume to skin tissues and/or tissues underneath the skin. A dermal filler is generally formulated in the form of an aqueous gel, i.e. a hydrogel. The term "gel", as used herein, broadly refers to a material having fluidity at room temperature between that of a liquid and solid. A hydrogel of the present invention generally comprises polymers (i.e. FA-g-HA and/or HA) and an aqueous solution (e.g., a physiologically acceptable carrier fluid, particularly a non-pyrogenic isotonic buffer, more particularly a physiological saline solution or a buffered physiological saline solution).

It was unexpectedly found that the fatty acid-grafted hyaluronic acid (FA-g-HA) according to the present invention spontaneously forms microspheres in aqueous solution by self-aggregation, which can be formulated as a microsphere-based dermal filler composition having advantageous properties as mentioned hereinbefore, such as low injection force but at the same time a sufficiently high lifting capacity.

Such a microsphere-based dermal filler formulation is described in detail in connection with the fourth aspect of the present invention. The dermal filler formulation does not require any crosslinking agent and, thus, the use of a potentially harmful crosslinking agent can be avoided. This is advantageous since the commonly used BDDE (1,4-butanediol diglycidyl ether) crosslinking agent is generally considered toxic and found to be mutagenic in Drosophila. Therefore, the amount of BDDE in dermal fillers is to be limited to a one digit ppm range (preferably <2 ppm) by appropriate purification procedures in order to meet regulatory requirements.

Furthermore, it was also unexpectedly found that the fatty acid-grafted hyaluronic acid (FA-g-HA) is excellent for use as a lubricant (or "lubrication phase") in a HA-based dermal filler, in particular in a crosslinked HA-based dermal filler, and more particularly in a BDDE-crosslinked dermal filler. A sterile crosslinked hyaluronic acid-based dermal filler formulation comprising FA-g-HA is described in detail in connection with the sixth aspect of the present invention.

In addition to the advantages mentioned above in the Summary section, the microspheres of FA-g-HA can be formulated as carriers for poorly water soluble substances, such as oils, triglycerides and fatty acids. This is, the microspheres of FA-g-HA contain encapsulated water soluble substances that are released at the site of injection. In fact, due to the encapsulation of the poorly water soluble substances, and the steric hindrance of the optionally present crosslinked HA, they are released in a delayed manner at the site of injection which results in a prolonged effect.

In particular, the use of microspheres with encapsulated oils, triglycerides or fatty acids was found to result in the uptake of fatty acids by adipocytes and the storage inside these cells, resulting in an increase in cell volume, or consumption of the fatty acids by the adipocytes, thereby stimulating/inducing them. In other words, the volume-creating ability of crosslinked HA and the volume increase by adipocytes act in concert to achieve a more pronounced and persistent lifting effect. Thus, the present invention provides additional benefits, i.e. a nutritional effect on cells.

According to a fourth aspect of the present invention, there is provided a sterile microsphere-based dermal filler formulation, comprising microspheres of fatty acid-grafted hyaluronic acid according to claims 4 and 5.

A "microsphere" in the context of the present invention means a particle, in particular substantially spherical particle, of fatty acid-grafted hyaluronic acid (FA-g-HA). In aqueous medium, the lipophilic fatty acid groups are thought to form the interior of the microsphere, while the more hydrophilic hyaluronic acid backbone of the FA-g-HA are oriented to the outside and exposed to the aqueous medium. Within the present invention, the term "microsphere" includes microspheres that consists of FA-g-HA, i.e. microspheres that do not, or essentially do not, comprise any other substances than FA-g-HA ("empty" microsphere) as well as microspheres containing, in its interior, encapsulated substances such as poorly water soluble or hydrophobic substances (e.g., oils, triglycerides, fatty acids).

Preferably, the microspheres of fatty acid-grafted hyaluronic acid have a particle size distribution characterized by a D50 value of 0.1 $\mu$m to 2 $\mu$m. Particularly preferred, the D50 value is from 0.2 $\mu$m to 1.5 $\mu$m or from 0.2 $\mu$m to 1.0 $\mu$m, and more preferably from 0.3 $\mu$m to 0.9 $\mu$m, from 0.4 $\mu$m to 0.8 $\mu$m or from 0.5 $\mu$m to 0.7 $\mu$m. The D50 value is defined as the particle diameter, at which 50% by number of the particles have a smaller diameter than the diameter, which corresponds to the D50 value, and 50% by number of the particles have a larger diameter than the D50 value.

The determination of the particle size can be carried out by nanoparticle tracking analysis (NTA analysis). Light scattering microparticles of described size have characteristically brownian motion in aqueous solution, which means that microparticle movement is reverse proportional to its diameter. Based on microparticle tracking over time, a precise determination of particle size distribution can be obtained.

Within the framework of the present invention, it is desirable that the microspheres have a narrow particle size distribution. The particle size distribution can be adjusted by subjecting a microsphere containing aqueous formulation to a high pressure homogenization (HPH) treatment, as described in connection with the process according to the fifth aspect of the present invention.

A desirable narrow particle size distribution of the microparticles is characterized by the values of D10, D50 and D90. Specifically, in accordance with the present invention, D10 is preferably above 0.1 $\mu$m, and D90 is preferably equal to or less than 1.0 $\mu$m.

The total concentration of the fatty acid-grafted hyaluronic acid in the microsphere-based dermal filler formulation typically ranges from 1 mg/ml to 100 mg/ml or from 2 mg/ml to 70 mg/ml, in particular from 3 mg/ml to 50 mg/ml, from 4 mg/ml to 40 mg/ml or from 5 mg/ml to 30 mg/ml.

In accordance with the present invention, the microspheres of fatty acid-grafted hyaluronic acid may consist only of FA-g-HA. However, the microspheres of FA-g-HA may also comprise encapsulated substances such as oils, triglycerides and fatty acids. In this case, the total concentration of encapsulated substance is usually from 0.01 to 30 wt.%, from 0.1 to 20 wt.% or from 1.0 to 15 wt.%, in particular from 2 to 10 wt.% or from 1 to 5 wt.%.

Preferably, the microspheres of FA-g-HA and encapsulated substance, e.g. a poorly water soluble substance such as oil, triglycerides and fatty acids, have a particle size distribution characterized by a D50 value of 1.5 μm to 50 μm. Particularly preferred, the D50 value is from 2.0 μm to 30 μm or from 3.0 μm to 20 μm, and more preferably from 5.0 μm to 11.0 μm, from 6.0 μm to 10.0 μm or from 6.0 μm to 8.0 μm. The D50 value is defined as the particle diameter, at which 50% by number of the particles have a smaller diameter than the diameter, which corresponds to the D50 value, and 50% by number of the particles have a larger diameter than the D50 value. The determination of the particle size can be carried out by laser diffractive analysis.

Further substances may also be comprised in the microsphere-based hydrogel formulation (e.g. polyols, antioxidants etc.) as described below in connection with the sixth aspect of the present invention.

In accordance with the present invention, the microsphere-based hydrogel formulation of fatty acid-grafted hyaluronic acid is sterile. Sterilization may be accomplished by subjecting the formulation to moist heat, e.g. by autoclaving. Conveniently, the formulation may be filled into a syringe, followed by sterilizing the prefilled syringe by moist heat, e.g. autoclaving. For example, the hydrogel formulation may be autoclaved at a temperature of 121°C to 127°C for 3 to 10 minutes.

Furthermore, the sterile microsphere-based hydrogel formulation is injectable. This means that the formulation is suitable for injection into the skin or other tissue in order to bring the hydrogel formulation to the desired target site. In particular, the hydrogel formulation may have an injection force, as measured through a needle of 30G½ at a rate of 12.5 mm/min using a 1 ml glass syringe, of 1 to 50 N, preferably 2 to 40 N or 3 to 30 N, more preferably 4 to 25 N, 5 to 20 N, 6 to 15 N, 7 to 12 N or 8 to 10 N. The microsphere-based dermal filler formulation of the present invention generally comprises an aqueous solvent, preferably a buffered aqueous solution, more preferably a phosphate-buffer or a phosphate-citrate-buffer, and optionally an osmolality adjuster, particularly sodium chloride, in particular a saline solution or a saline phosphate buffer. The pH of the microsphere-based dermal filler formulation is generally in the range of 6.5 to 7.5, 6.5 to 7.4 or 6.5 to 7.1, or may be in the range of 6.8 to 7.4.

The microsphere-based hydrogel formulation of fatty acid-grafted hyaluronic acid has preferably a complex viscosity at 1 Hz of 2 Pa·s to 80 Pa·s, preferably of 5 Pa·s to 70 Pa·s or 10 Pa·s to 60 Pa·s, more preferably of 15 Pa·s to 60 Pa·s or 20 Pa·s to 50 Pa·s. The complex viscosity may be measured using a commercially available rheometer. Furthermore, the hydrogel formulation preferably has an elastic modulus (G') at 1 Hz of 10 Pa to 500 Pa, more preferably 50 Pa to 500 Pa or 100 Pa to 400 Pa, and most preferably 150 to 500 Pa, 200 to 450 Pa, 250 to 400 Pa or 300 to 350 Pa. In addition, the hydrogel formulation preferably has a tan delta, defined as the ratio of viscous modulus (G") to elastic modulus (G') at 1 Hz, of 0.5 or higher, preferably 0.6 or higher or 0.8 or higher, more preferably 1.0 or higher, 1.1 or higher, 1.2 or higher, 1.3 or higher, 1.4 or higher, or 1.5 or higher.

According to a fifth aspect, the present invention relates to a process for the preparation of a sterile microsphere-based dermal filler formulation according to the fourth aspect of the present invention, comprising the steps of:

   (A) contacting a fatty acid-grafted hyaluronic acid having a hyaluronic acid backbone of 1,250 to 12,500 repeating disaccharide units, wherein the fatty acid is a C6-C22 fatty acid, with an aqueous solution to obtain a microsphere-based formulation of fatty acid-grafted hyaluronic acid,
   (B) optionally subjecting the microsphere-based formulation of fatty acid-grafted hyaluronic acid to a high pressure homogenization (HPH) treatment, and
   (C) sterilizing the optionally HPH-treated, microsphere-based formulation to obtain a sterile microsphere-based dermal filler formulation.

In step (A), the aqueous formulation may be a formulation that is obtained in the preparation process of the fatty acid-grafted hyaluronic acid described above without a step of isolating the FA-g-HA in solid form. Alternatively, the aqueous formulation of FA-g-HA may be prepared by adding an aqueous solution to FA-g-HA in solid, e.g. freeze-dried, form.

Optional step (B) results in a narrower particle size distribution, which generally has a positive impact on the properties of the final dermal filler product. Step (B) may be carried out in a high pressure homogenizer. A high pressure homogenizer is a device which applies a pressure to a liquid while feeding this phase through a homogenization opening. In accordance with the present invention, the homogenization step may be carried out at room temperature (20-25°C) and a pressure of 1 to 10 bar, such as at 20°C and 1 bar.

In step (C), sterilization by moist heat, in particular by autoclaving, is used for sterilization. For example, the formulation may be autoclaved at a temperature of 121°C to 127°C for 3 to 10 minutes. Furthermore, before sterilization, but after step (A) or (B), the aqueous formulation of a fatty acid-grafted hyaluronic acid is preferably filled into a syringe. The prefilled syringe may then be sterilized in step (C).

According to a sixth aspect of the present invention, there is provided a sterile crosslinked hyaluronic acid-based dermal filler formulation comprising hyaluronic acid crosslinked with a crosslinking agent, in particular 1,4-butanediol diglycidyl ether (BDDE), and a fatty acid-grafted hyaluronic acid according to claim 7.

The fatty acid-grafted hyaluronic acid (HA-g-FA) is not crosslinked and used as a separate phase that is added to the (pre-existing) crosslinked HA phase. The HA-g-FA may be characterized as in claim 7.

In accordance with the present invention, the HA-g-FA is present in the sterile crosslinked hyaluronic acid-based dermal filler composition in an amount of 0.0001 wt.% to 20.0 wt.%, 0.001 wt.% to 10.0 wt.%, 0.01 wt.% to 5.0 wt.%, 0.05 wt.% to 2.0 wt.%, or 0.1 wt.% to 1 wt.%.

Generally, the HA-g-FA is present in the dermal filler formulation in the form of microspheres, and the particle size distribution is defined as described in connection with the fourth aspect of the present invention. The microspheres of fatty acid-grafted hyaluronic acid preferably have a particle size distribution characterized by a D50 value of 0.1 $\mu$m to 2 $\mu$m. Particularly preferred, the D50 value is from 0.2 $\mu$m to 1.5 $\mu$m or from 0.2 $\mu$m to 1.0 $\mu$m, and more preferably from 0.3 $\mu$m to 0.9 $\mu$m, from 0.4 $\mu$m to 0.8 $\mu$m or from 0.5 $\mu$m to 0.7 $\mu$m. The D50 value is defined as the particle diameter, at which 50% by number of the particles have a smaller diameter than the diameter, which corresponds to the D50 value, and 50% by number of the particles have a larger diameter than the D50 value.

Furthermore, the microspheres may contain encapsulated substances, in particular poorly water soluble substances like oils, triglycerides and fatty acids, as described herein above. In this case, the total concentration of encapsulated substance in the dermal filler formulation is usually from 0.01 to 30 wt.%, from 0.1 to 20 wt.% or from 1.0 to 15 wt.%, in particular from 2 to 10 wt.% or from 1 to 5 wt.%. Preferably, the microspheres of FA-g-HA and encapsulated substance, e.g. a poorly water soluble substance such as oil, triglycerides and fatty acids, have a particle size distribution characterized by a D50 value of 1.5 $\mu$m to 50 $\mu$m. Particularly preferred, the D50 value is from 2.0 $\mu$m to 30 $\mu$m or from 3.0 $\mu$m to 20 $\mu$m, and more preferably from 5.0 $\mu$m to 11.0 $\mu$m, from 6.0 $\mu$m to 10.0 $\mu$m or from 6.0 $\mu$m to 8.0 $\mu$m.

The crosslinked HA may be present in the composition in a concentration of from 1 mg/ml to 60 mg/ml or from 2 mg/ml to 50 mg/ml, preferably from 3 mg/ml to 40 mg/ml or from 5 mg/ml to 35 mg/ml, more preferably from 10 mg/ml to 30 mg/ml or from 15 mg/ml to 28 mg/ml, and most preferably from 20 mg/ml to 25 mg/ml.

Furthermore, the HA preferably has an average molecular mass of $0.5 \times 10^6$ Da to $4.0 \times 10^6$ Da, in particular $1.0 \times 10^6$ Da to $3.0 \times 10^6$ Da or $1.5 \times 10^6$ Da to $2.5 \times 10^6$ Da. The average molecular mass can be determined as described herein above.

The crosslinked HA is preferably BDDE (1,4-butanediol diglycidyl ether)-crosslinked. The BDDE-crosslinked hyaluronic acid may have a degree of modification, expressed as the ratio of the sum of mono- and double-linked BDDE-cross-linkers to the sum of hyaluronic acid disaccharide units, of 0.5% to 25%, preferably 1.0% to 15%, more preferably 2.0% to 10%, and most preferably 3.0% to 8.0% or 4.0% to 7%.

The degree of modification may be determined by nuclear magnetic resonance (NMR) in accordance with methods known in the art (Edsman et al., Gel Properties of Hyaluronic Acid Dermal Fillers, Dermatol. Surg. 2012, 38:1170-1179; Guarise et al., SEC determination of cross-link efficiency in hyaluronan fillers, Carbohydrate Polymers 2012, 88:428-434; Kenne et al., Modification and crosslinking parameters in hyaluronic acid hydrogels - Definitions and analytical methods, Carbohydrate Polymers 2013, 91:410-418). The dialyzed and sterilized gels are degraded before conducting the NMR measurement. The degradation can be performed by chondroitinase AC (Edsman *et al., supra;* Kenne *et al., supra),* NaOH (Guarise *et al., supra),* addition of hyaluronidase (e.g., 150 U ovine hyaluronidase to 1 g of gel) or by incubation at 90°C for at least 35 h. The obtained solutions are then lyophilized, dissolved in $D_2O$, and well homogenized.

The NMR measurement may be performed at, e.g., 500 MHz, at a pulse of 20 degree with several repetitions at ambient temperature to receive a spectrum with appropriate resolution. In accordance with the literature, the degree of modification (MoD) is assessed by calculating the ratio of the N-acetyl signals of HA to the methylene signals of BDDE. For N-acetyl of HA, the critical signals are located at about 2.0 ppm and at about 1.6 ppm for BDDE when solubilized in $D_2O$. In order to calculate the degree of modification, the integral values were identified and the ratio of protons of 3H of N-acetyl ($CH_3$) to 4H of methylene ($CH_2CH_2$) needs to be taken in account, in accordance with the literature (Edsman *et al., supra,* and Kenne *et al., supra*).

Otherwise, the crosslinked hyaluronic acid (crosslinked HA) is not limited in any particular way and may include crosslinked HA prepared from a single HA or from two or more HAs that differ in their molecular weight (see, e.g., US 2010/0316683 A1 or WO 2013/185934 A1). For example, the crosslinked HA may be made by crosslinking two different HAs, wherein the first hyaluronic acid may have an average molecular mass of $1.0 \times 10^6$ Da to $1.5 \times 10^6$ Da, and the second hyaluronic acid may have an average molecular mass of $2.8 \times 10^6$ Da to $3.2 \times 10^6$ Da. "Polydensified" gels with varying degrees of crosslinking within the gel are also within the scope of the present invention.

Furthermore, the microsphere-based hydrogel formulation according to the fourth aspect of the present invention as well as the crosslinked hyaluronic acid-based dermal filler formulation according to the sixth aspect of the present invention may optionally comprise a local anesthetic agent such as lidocaine. The local anesthetic, in particular lidocaine, may be present in the formulation in a concentration of, for example, 0.05 wt.% to 5.0 wt.%, 0.1 wt.% to 4.0 wt.%, 0.2 wt.% to 3.0 wt.%, 0.3 wt.% to 2.0 wt.%, or 0.4 wt.% to 1.0 wt.%. However, in one embodiment, the microsphere-based hydrogel formulation of fatty acid-grafted hyaluronic acid does not contain an anesthetic agent or a local anesthetic agent or lidocaine (e.g., lidocaine hydrochloride).

In addition, the microsphere-based hydrogel formulation according to the fourth aspect of the present invention as well as the crosslinked hyaluronic acid-based dermal filler formulation according to the sixth aspect of the present invention may further comprise additional substances like polyols, vitamins, amino acids, metals, antioxidants, hydroxyapatite particles, and mineral salts (e.g., a Zn salt), either in combination with or without a local anesthetic agent like lidocaine.

Suitable polyols for use herein include, but are not limited to, glycerol, mannitol, sorbitol, propylene glycol, erythritol, xylitol, maltitol, and lactitol. Particularly suitable for use herein is mannitol and glycerol. Further, the polyol is preferably glycol, optionally in combination with one or more of the aforementioned polyol compounds, in particular mannitol. The polyol(s) may, for example, be included in the dermal filler composition in a concentration of 0.1% to 25% or 1% to 20% or 2% to 15% volume/volume, particularly in a concentration of 5% to 10% volume/volume.

Suitable vitamins include vitamin C, vitamin E and vitamins of the B group, i.e. one or more of $B_1$, $B_2$, $B_3$, $B_5$, $B_6$, $B_7$, $B_9$ and $B_{12}$ vitamins. The concentration of vitamin C or of vitamin E is preferably from about 0.01 mg/ml to about 10.0 mg/ml, more preferably from about 0.1 mg/ml to about 5.0 mg/ml, and the total concentration of the vitamins of the B group is preferably from about 0.01 mg/ml to about 10.0 mg/ml, more preferably from about 0.1 mg/ml to about 5.0 mg/ml. The vitamins may be present to stimulate and maintain cellular metabolism and, thus, to promote collagen production. Particularly preferred for use here is vitamin C, vitamin E and vitamin $B_6$.

It is further contemplated herein that the dermal filler compositions according to the fourth and sixth aspect of the present invention may include non-crosslinked HA ("free" HA). In particular, the dermal filler composition may further comprise 0.001% to 15%, in particular 1% to 10% volume/volume non-crosslinked hyaluronic acid. The molecular weight of said non-crosslinked hyaluronic acid is preferably between $3.0 \times 10^5$ Da and $4.0 \times 10^6$ Da, in particular between $1.0 \times 10^6$ Da and $3.0 \times 10^6$ Da.

Preferably, the dermal filler composition of the present invention lacks any crosslinked polymers other than crosslinked HA. Further preferably, the dermal filler composition of the present invention lacks any uncrosslinked polymers other than the FA-g-HA. Yet further preferably, the dermal filler composition of the present invention lacks any crosslinked polymers other than crosslinked HA and lacks any uncrosslinked polymers other than the FA-g-HA.

According to a seventh aspect of the present invention, there is provided a process for the preparation of a sterile crosslinked hyaluronic acid-based dermal filler formulation according to the sixth aspect of the present invention, comprising the steps of:

(i) providing a fatty acid-grafted hyaluronic acid having a hyaluronic acid backbone of 1,250 to 12,500 repeating disaccharide units, wherein the fatty acid is a C6-C22 fatty acid,
(ii) providing a crosslinked hyaluronic acid,
(iii) combining the fatty acid-grafted hyaluronic acid and the crosslinked hyaluronic acid to obtain a crosslinked hyaluronic acid-based dermal filler formulation, and
(iv) sterilizing the crosslinked hyaluronic acid-based dermal filler formulation to obtain a sterile crosslinked hyaluronic acid-based dermal filler formulation.

The FA-g-HA is added in uncrosslinked form, in particular in the form of a solution, to a pre-formed crosslinked HA gel, in particular a BDDE cross-linked HA gel, to obtain a dermal filler material having improved properties as compared to the crosslinked HA gel. The FA-g-HA acts as a lubricant and imparts additional benefits to the crosslinked HA-based gel.

Sterilization is preferably carried out by moist heat, in particular by autoclaving, as described herein above.

According to an eighth aspect of the present invention, there is provided a prefilled syringe, comprising a sterile dermal filler composition according to claims 4, 5, 7, 8 and 9.

These syringes are ready-to-use and therefore particularly convenient for use by the practitioner.

According to a ninth aspect, the present invention relates to the use of a sterile dermal filler formulation according to claims 4, 5, 7, 8 and 9 for cosmetic treatments, in particular for augmenting or filling of wrinkles or fine lines of the skin (e.g., nasolabial folds, marionette lines, chin folds, lower jawlines, oral commissure, and the like), filling cutaneous depressions, masking scars, increasing the volume of the lips, augmenting cheeks, nose corrections, and/or improve skin hydration and skin texture.

Also disclosed but not part of the claimed invention, is a method for augmenting or filling soft tissues, comprising administering to a subject in need thereof an effective amount of a sterile dermal filler formulation according to the fourth or sixth aspect of the present invention.

The dermal filler composition of the present invention is generally administered by injection, such as by subcutaneous or intradermal injection. For example, the composition may be intradermally or subcutaneously injected using the serial puncture technique.

The term "effective amount" refers to the amount of the (injectable) soft tissue filler composition sufficient to effect beneficial or desired cosmetic (aesthetic) results. A "subject" in the sense of the present invention is any individual or patient, usually a human, in need of the treatment of a particular condition or disease.

The present invention will now be further illustrated by the following, nonlimiting examples.

## EXAMPLES

The examples provided below illustrate the preparation of fatty acid-grafted hyaluronic acid (FA-g-HA) dermal fillers according to the present invention. The examples further show that the FA-g-HA dermal fillers according to the present invention have lifting capacity and stability without compromising the ease of injection and, thus, represent a promising approach for various aesthetic applications.

The measurement methods used herein to characterize a FA-g-HA dermal filler according to the present invention and comparative or control formulations are described below.

*Rheological measurements*

Rheological measurements were carried out using an Anton Paar MCR 302 Rheometer equipped with a cone of 1°, 50 mm diameter. The measurements were performed at a shear deformation of 0.1% and a frequency from 0.1 to 10 Hz with a constant gap of 0.1 mm at 25°C.

*GPC measurements*

The following analytical conditions were used for the GPC measurement. Eluent: PBS buffer pH 7.4 in deionized water; Precolumn: PSS Suprema, 10 $\mu$m, Guard, 8.0 mm x 50 mm; columns: 3x PSS Suprema, 10 $\mu$m, UltraHigh, 8.0 mm x 300 mm; Pump: Agilent-SECcurity pump; Flow: 0.5 ml; Injection system: Agilent-SECcurity Autosampler, temperature: 35 °C, detectors: PSS-SECcurity DRI, PSS SLD 7000 multiangle laser light scattering (MALLS); Evaluation: PSS - WinGPC UniChrom Version 8.2. Sample preparation included weighing two samples using an analytical balance and adding a calculated volume of eluent (also weighed). The final concentrations were adjusted to 1 g/L and 1.5 g/L, respectively. The samples were dissolved at room temperature and filtered through a single-use filter (pore size: 1 $\mu$m) before measurement.

*Extrusion force measurements*

The extrusion force was measured using a Texture Analyser (TA-XT plus, Stable Micro Systems, Haslemere, Surrey, UK) through a TSK needle of 30G½ at a rate of 12.5 mm/min using a standard glass syringe (BD, 1 mL).

*Nuclear Magnetic Resonance ($^1$H-NMR) spectroscopy studies*

Samples were measured in duplicate at room temperature under a frequency of 500 MHz with a spectral width of 10 kHz and 200 repetitions. Sample preparation included weighing 25 mg FA-g-HA using an analytical balance and adding a calculated volume of deuterated water (1 mL) containing hyaluronase (300 U/mL), resulting in a final concentration of FA-g-HA of 25 mg/mL. The sample was then vortexed and placed in an oven at 40°C for 12 hours to degrade the viscous solution.

Example 1

*Synthesis of caprylic acid-grafted HA (C8-g-HA)*

*Synthesis of C8-g-HA*

Mw (native) HA = 1.8x10$^6$ Da; fatty acid (FA) = caprylic acid (C8), molar ratio (HA:FA) = 1:4, G'$_{1Hz}$ (Pa) (non-sterile) = 142.0 Pa, DoM= 4 %

*Preparation of highly surfaced solid HA*

Native sodium hyaluronate (NaHA) fibers (10 g; Mw = 1.8 MDa) were dispersed in 1 L deionized water using an Ultra-Turrax (25.000 rpm, 5 minutes), followed by resting for 5 hours or overnight. The air bubble-free HA gel was then frozen (8 hours at $\leq$ -10°C). In order to remove water, the material was freeze-dried for 7-14 days, obtaining a sponge-like porous material.

*Coupling reaction*

The freeze-dried HA (1 g, n = 2.64 $\mu$mol) was then weighed into a 100 ml beaker. 50 mL of n-pentane was added. The resulting suspension was stirred continuously while adding caprylic acid chloride ($C_8$ chloride) in defined amounts (4 $n_{(fatty\ acid\ chloride)}$ x $n_{(HA)}$). As an alternative, said resulting suspension may also be stirred continuously while adding fatty acid (C8) anhydride in defined amounts (4 $n_{(fatty\ acid\ anhydride)}$ x $n_{(HA)}$). Then, the suspension was stirred at room temperature for 8 hours. After stirring, the solid material was separated from the solvent by filtration and washed three times with n-pentane to remove uncoupled oil-soluble components, thereby obtaining a solid material.

50 mL of a buffer solution (phosphate buffer, $KH_2PO_4$/$Na_2HPO_4$, 5-50 g/L, pH 7) were then added to the solid material and stirred for about 8-12 hours. The resulting precipitate was then removed from the aqueous supernatant, three times washed with iso-propanol and again dissolved in 10 mL deionized water. Complete swelling for further 10 h at 2-8°C of the resulting material yielded an aqueous dispersion of C8-g-HA microglobuli/micells. The dispersed product was then freeze-dried to obtain a solid C8-g-HA material in the form of a sponge-like porous material.

Alternatively, if fatty acid (C8) anhydrides were used instead of fatty acid chlorides (see above), 50 mL of deionized water and n-pentane were added to the solid material obtained after washing with n-pentane and stirred for about 8-12 hours. The upper n-pentane phase was then removed, while the aqueous phase was freeze-dried to obtain a solid C8-g-HA material in the form of a sponge-like porous material.

The obtained C8-g-HA was characterized by rheological and extrusion force measurements (see Example 3), accelerated stability testing (see Example 4), particle size determination (see Example 5).

EXAMPLE 2

*Synthesis of palmitic acid-grafted HA (C16-g-HA)*

Palmitic acid-grafted HA (C16-g-HA) was prepared in the same manner as described in Example 1, except that palmitic acid chloride was used instead of caprylic acid chloride. The obtained C16-g-HA exhibited a viscous modulus $G'_{1Hz}$ (Pa) (non-sterile) of 17.1 Pa. This Example shows the successful grafting of much longer fatty acids than caprylic acid to obtain a medium chain fatty acid grafted HA.

EXAMPLE 3

*Rheological properties and extrusion force of C8-g-HA of the invention in comparison to commercial HA-C6, native HA and a commercial HA-based dermal filler*

The inventive C8-g-HA prepared in Example 1 was characterized in terms of its rheological properties and extrusion force as described above. As a comparison, a caproic acid (C6)-acylated derivative of HA that is commercially available from Contipro a.s., Czech Republic (referred to as "HA-C6" in the following), and native HA as well as a commercial HA-based dermal filler (Belotero®; Merz Aesthetics) were examined with regard to their rheological properties and extrusion force.

Sample preparation for the C8-g-HA (1.8 MDa) material prepared in Example 1 included weighing 270 mg of lyophilized material (4% DoM; equal to 260 mg native HA) using an analytical balance and adding a defined volume of 10 mL saline solution (NaCl = 0.9 wt.%).

Sample preparation for HA-C6 (Contipro a.s.; 240 kDa) included weighing 430 mg of lyophilized material (40% DoM; equal to 260 mg native HA) using an analytical balance and adding a defined volume of 10 mL saline solution (NaCl = 0.9 wt.%).

Sample preparation for the native HA (3.5 MDa) included weighing 260 mg of lyophilized HA using an analytical balance and adding a defined volume of 10 mL saline solution (NaCl = 0.9 wt.%).

Each of the samples (C8-g-HA, HA-C6 Contipro, and native HA) had a concentration of 26 mg/mL HA. After resting overnight, the samples were sterilized by autoclaving at 131°C for 10 seconds.

The Belotero® dermal filler (Merz Aesthetics) is a BDDE-crosslinked HA gel with a HA concentration of 26 mg/ml and was used as commercially available.

**Table 1.** Rheological properties of sterile C8-g-HA of the present invention in comparison to sterile HA-C6, sterile native HA, and Belotero®

| Example | $M_w$ (HA) (Da) | Fatty acid | Extrusion force (N) | Complex viscosity$_{1\,Hz}$ (Pa·s) | G'$_{1Hz}$ (Pa) | G"$_{1Hz}$ (Pa) | tan$\delta_{1Hz}$ | G' = G"$_{1Hz}$ (gelling pt.) (Pa) |
|---|---|---|---|---|---|---|---|---|
| HA-C8 (inventive) | $1.8 \times 10^6$ | Capryl acid (C8:0) | 8.1 | 9.2 | 35.4 | 45.3 | 1.3 | 75 (0.1% deformation, 3Hz) |
| HA-C6 (Contipro) comparison | $0.24 \times 10^6$ | Caproic acid (C6:0) | 6.6 | 2.0 | 12.3 | 1.6 | 0.1 | 6 (140% deformation, 1Hz) |
| HA (native) (comparison) | $3.5 \times 10^6$ | -- | 8.1 | 0.8 | 0.5 | 4.8 | 9.1 | n.d. |
| Belotero® (comparison) | n.s. | -- | 23.4 | 41.3 | 252 | 59.1 | 0.2 | 525 (517% deformation, 1Hz) |

As can be seen from Table 1, the inventive C8-g-HA shows a desirably low extrusion force of 8.1 N, which is comparable to that of the commercially available HA-C6 (despite the much lower molecular weight of HA) and essentially identical to that of HA native with a molecular weight of 3.5 MDa. In contrast, the dermal filler Belotero® has a much higher extrusion force. In other words, the extrusion force required to inject this dermal filler through a fine needle is significantly higher. The extrusion force, however, is of key importance in clinical practice since a consistent and even extrusion force affords control and precision during an injection, thereby facilitating easier and more accurate treatments.

EXAMPLE 4

*Accelerated stability testing*

The stability of C8-g-HA was determined using an accelerated stability testing method at 25°C and 60% relative humidity and at 40°C and 75% relative humidity. The results are shown in Table 2.

**Table 2.** Stability of sterile HA-C$_8$ (30 mg/ml) as determined by accelerated stability testing at 25°C/60% RH and 40°C/75% RH

| Time (t) | Accelerated storage conditions | Complex viscosity$_{1Hz}$ (Pa·s) | G'$_{1Hz}$ (Pa) | G"$_{1Hz}$ (Pa) | Tan$\delta_{1Hz}$ | G' decrease (%) |
|---|---|---|---|---|---|---|
| 0 weeks (none sterile) | -- | 26.6 | 142 | 88.2 | 0.6 | -- |
| 0 weeks (initial) | -- | 9.2 | 35.4 | 45.3 | 1.3 | -- |
| 6 weeks | 25°C/60% RH | 9.1 | 34.5 | 45.9 | 1.3 | 3 |
| 6 weeks | 40°C/75% RH | 6.7 | 22.5 | 35.6 | 1.6 | 37 |
| 8 weeks | 25°C/60% RH | 8.5 | 31.8 | 43.3 | 1.4 | 10 |
| 8 weeks | 40°C/75% RH | 6.0 | 19.4 | 32.6 | 1.7 | 45 |
| 12 weeks | 25°C/60% RH | 9.4 | 35.5 | 46.0 | 1.3 | 0 |
| 12 weeks | 40°C/75% RH | 5.1 | 15.1 | 28.6 | 1.9 | 58 |
| 20 weeks | 25°C/60% RH | 8 | 28.8 | 41.2 | 1.4 | 19 |
| 20 weeks | 40°C/75% RH | 4.12 | 10.7 | 23.6 | 2.2 | 70 |

EXAMPLE 5

*Encapsulation of oil (middle chain triglycerides; MCT)*

This example investigates the encapsulation of middle-chain triglyceride (MCT) oil as a lipophilic component into FA-g-HA microglobuli. To this extent, an MCT (middle chain triglycerides) oil in 0.9% NaCl solution was first subjected to high pressure homogenization (HPH). The pressure applied was 10 bar but may range from 10 to 1,500 bar. The resulting emulsion contained narrow-sized MCT oil particles with a concentration of 20 mg/mL in saline sodium chloride solution.

In a second step, the emulsion was mixed with a FA-g-HA solution (52 mg/ml) in a 1:1 volume ratio, for example in a syringe or a mixing device, so as to receive a final concentration after mixing of 26 mg/mL HA. The exemplary FA-g-HA material used was sterile C8-g-HA (Mw (HA) = 1.8 MDa, DoM = 4%, 26 mg/ml HA) as prepared in Example 1. As a control, sterile and non-sterile C6-g-HA (Contipro a.s.; Mw (HA) = 240 kDa, DoM = 40%, 26 mg/ml HA) in saline sodium chloride solution were used. The mixture was then left over night at refrigerator temperature (about 4°C). As a result, the FA-g-HA encloses with its lipophilic part the oil droplets in the emulsion leading to the formation of a stabilized autoclavable emulsion.

SEC experiments showed that high pressure homogenization (HPH) at a pressure of 10 bar does not affect HA chain breakage, as indicated by essentially identical Mw (kDa) and Mn (kDa) values of HA (Mw = $1.8 \times 10^6$ Da) after repeated cycles of HPH (results not shown).

Whilst it is possible to subject a mixture of the encapsulating material (e.g., the MCT) and the FA-g-HA in a suitable medium to a HPH treatment, it was found that separating the process into two steps, i.e. first preparing an emulsion of the encapsulating material and then mixing the emulsion with the FA-g-HA, results in an emulsion with a more narrow particle size distribution.

EXAMPLE 6

*Particle size distribution*

The particle size distribution of the oil emulsions obtained in Example 5, was measured using laser diffractive analysis. The results are shown in Table 3.

**Table 3.** Particle size distribution

| Sample | Number average diameter D[3,2] ($\mu$m) | Volume average diameter D[4,3] ($\mu$m) | Median particle size | | |
|---|---|---|---|---|---|
| | | | D10 ($\mu$m) | D50 ($\mu$m) | D90 ($\mu$m) |
| Sterile C8-g-HA (according to invention) (Mw HA = 1.8 MDa, DoM = 4%, 26 mg/ml HA) | -- | -- | 0.38 | 0.65 | 0.90 |
| Non-sterile HA-C6 (Contipro) (Mw HA = 240 kDa, DoM = 40%, 30 mg/ml HA, MCT 10 mg/ml) | 4.2 | 7.0 | 1.8 | 6.7 | 12.4 |
| Sterile HA-C6 (Contipro) (Mw HA = 240 kDa, DoM = 40%, 30 mg/ml HA, MCT 10 mg/ml) | 4.2 | 6.9 | 1.8 | 6.6 | 12.3 |

As can be seen from Table 3, the encapsulating process involving high pressure homogenization (HPH) and subsequent autoclavation results in a narrow particle size distribution in the sub-micron range. Furthermore, as is evident from a comparison of the non-sterile and sterile HA-C6 samples, autoclavation does not affect particle size distribution.

EXAMPLE 7

*Nutritional effects on adipocytes*

The FA-g-HA of the present invention was studied with regard to its usability as a carrier for triglyceride nutrition of adipocytes. It was found that contacting adipocytes with microspheres of FA-g-HA encapsulating triglycerides results in a marked increase of the measured glycerol compound in the adipocytes (results not shown). This demonstrates beneficial

EP 3 813 895 B1

nutritional effects on cells by the FA-g-HA of the present invention.

**Claims**

1. A process for the preparation of a fatty acid-grafted hyaluronic acid having a hyaluronic acid backbone of 1,250 to 12,500 repeating disaccharide units, wherein the fatty acid is a C6-C22 fatty acid, comprising the steps of:

   (a) dispersing hyaluronic acid in an organic solvent, in particular a non-polar solvent, to obtain an organic dispersion,
   (b) adding fatty acid chlorides to the organic dispersion,
   (c) allowing the fatty acid chlorides to esterify with hyaluronic acid under reaction conditions sufficient to form a fatty acid-grafted hyaluronic acid, and
   (d) isolating the fatty acid-grafted hyaluronic acid,

   wherein the hyaluronic acid dispersed in step (a) is pretreated by dispersing native hyaluronic acid, particularly native hyaluronic acid fibers, in an aqueous solution, thereby obtaining an aqueous dispersion and freeze-drying the aqueous dispersion to produce a freeze-dried hyaluronic acid which is then dispersed in step (a).

2. Use of a fatty acid-grafted hyaluronic acid having a hyaluronic acid backbone of 1,250 to 12,500 repeating disaccharide units, wherein the fatty acid is a C6-C22 fatty acid, in the preparation of a dermal filler formulation.

3. The use of claim 2, wherein said fatty acid-grafted hyaluronic acid is added to a HA-based dermal filler to function as a lubricant.

4. A sterile microsphere-based dermal filler formulation, comprising microspheres of fatty acid-grafted hyaluronic acid, the fatty acid-grafted hyaluronic acid having a hyaluronic acid backbone of 1,250 to 12,500 repeating disaccharide units, wherein the fatty acid is a C6-C22 fatty acid.

5. The sterile microsphere-based dermal filler formulation of claim 4, wherein the microspheres of fatty acid-grafted hyaluronic acid have a particle size distribution **characterized by** a D50 value of 0.1 $\mu$m to 2.0 $\mu$m.

6. A process for the preparation of a sterile microsphere-based dermal filler formulation according to claim 4 or 5, comprising the steps of:

   (A) contacting a fatty acid-grafted hyaluronic acid having a hyaluronic acid backbone of 1,250 to 12,500 repeating disaccharide units, wherein the fatty acid is a C6-C22 fatty acid, with an aqueous solution to obtain a microsphere-based formulation of fatty acid-grafted hyaluronic acid,
   (B) optionally subjecting the microsphere-based formulation of fatty acid-grafted hyaluronic acid to a high pressure homogenization (HPH) treatment, and
   (C) sterilizing the optionally HPH-treated, microsphere-based formulation to obtain a sterile microsphere-based dermal filler formulation.

7. A sterile crosslinked hyaluronic acid-based dermal filler formulation, comprising hyaluronic acid crosslinked with a crosslinking agent, in particular 1,4-butanediol diglycidyl ether (BDDE), and a fatty acid-grafted hyaluronic acid having a hyaluronic acid backbone of 1,250 to 12,500 repeating disaccharide units, wherein the fatty acid is a C6-C22 fatty acid.

8. The sterile crosslinked hyaluronic acid-based dermal filler formulation of claim 7, wherein the crosslinked HA is present at a concentration of 1 mg/ml to 50 mg/ml and/or wherein the fatty acid-grafted hyaluronic acid is present at a concentration of 0.0001 wt.% to 20 wt. %.

9. The sterile dermal filler formulation of any one of claims 4, 5, 7 and 8, further comprising an anesthetic agent, in particular lidocaine, and/or one or more further substances selected from the group consisting of polyols, vitamins, amino acids, metals, antioxidants, hydroxyapatite particles, and mineral salts.

10. A process for the preparation of a sterile crosslinked hyaluronic acid-based dermal filler formulation according to any one of claims 7 to 9, comprising the steps of:

(i) providing a fatty acid-grafted hyaluronic acid having a hyaluronic acid backbone of 1,250 to 12,500 repeating disaccharide units, wherein the fatty acid is a C6-C22 fatty acid,

(ii) providing a crosslinked hyaluronic acid,

(iii) combining the fatty acid-grafted hyaluronic acid and the crosslinked hyaluronic acid to obtain a crosslinked hyaluronic acid-based dermal filler formulation, and

(iv) sterilizing the crosslinked hyaluronic acid-based dermal filler formulation to obtain a sterile crosslinked hyaluronic acid-based dermal filler formulation.

11. The process of any one of claims 1, 6 and 10, the use of claim 2 or 3, or the formulation of any one of claims 4, 5, 7, 8 and 9, wherein the fatty acid is a C8-C18 fatty acid, preferably a saturated or unsaturated, linear C6-C22 fatty acid, in particular a saturated or unsaturated, linear C8-C18 fatty acid.

12. The process of any one of claims 1, 6 and 10, the use of claim 2 or 3, or the formulation of any one of claims 4, 5, 7, 8 and 9, wherein the degree of modification of hyaluronic acid with fatty acid is 1% to 30%.

13. A prefilled syringe, comprising a sterile dermal filler composition according to any one of claims 4, 5, 7, 8 and 9.

14. Use of a sterile dermal filler formulation according to any one of claims 4, 5, 7, 8 and 9 for cosmetic treatments, in particular for augmenting or filling of wrinkles or fine lines of the skin, filling cutaneous depressions, masking scars, increasing the volume of the lips, augmenting cheeks, nose corrections, and/or improve skin hydration and skin texture.

**Patentansprüche**

1. Verfahren zur Herstellung einer fettsäuregepfropften Hyaluronsäure mit einem Hyaluronsäure-Grundgerüst aus 1250 bis 12500 sich wiederholenden Disaccharid-Einheiten, wobei die Fettsäure eine C6-C22-Fettsäure ist, umfassend die Schritte:

(a) Dispergieren von Hyaluronsäure in einem organischen Lösungsmittel, insbesondere einem unpolaren Lösungsmittel, um eine organische Dispersion zu erhalten,

(b) Zugeben von Fettsäurechloriden zu der organischen Dispersion,

(c) Ermöglichen, dass die Fettsäurechloride mit der Hyaluronsäure unter Reaktionsbedingungen verestern, die zur Bildung einer fettsäuregepfropften Hyaluronsäure ausreichen, und

(d) Isolieren der fettsäuregepfropften Hyaluronsäure,

wobei die in Schritt (a) dispergierte Hyaluronsäure durch Dispergieren nativer Hyaluronsäure, insbesondere nativer Hyaluronsäurefasern, in einer wässrigen Lösung vorbehandelt wird, wodurch eine wässrige Dispersion erhalten wird, und Gefriertrocknen der wässrigen Dispersion, um eine gefriergetrocknete Hyaluronsäure herzustellen, die dann in Schritt (a) dispergiert wird.

2. Verwendung einer fettsäuregepfropften Hyaluronsäure mit einem Hyaluronsäure-Grundgerüst aus 1 250 bis 12 500 sich wiederholenden Disaccharid-Einheiten, wobei die Fettsäure eine C6-C22-Fettsäure ist, bei der Herstellung einer Dermalfüllstoffformulierung.

3. Verwendung nach Anspruch 2, wobei die fettsäuregepfropfte Hyaluronsäure einem HA-basierten Dermalfüllstoff zugesetzt wird, um als Gleitmittel zu wirken.

4. Sterile Mikrosphären-basierte Dermalfüllstoffformulierung, umfassend Mikrosphären aus fettsäuregepfropfter Hyaluronsäure, wobei die fettsäuregepfropfte Hyaluronsäure ein Hyaluronsäure-Grundgerüst aus 1 250 bis 12 500 sich wiederholenden Disaccharid-Einheiten aufweist, wobei die Fettsäure eine C6-C22-Fettsäure ist.

5. Sterile Mikrosphären-basierte Dermalfüllstoffformulierung nach Anspruch 4, wobei die Mikrosphären aus fettsäuregepfropfter Hyaluronsäure eine durch einen D50-Wert von $0,1\ \mu m$ bis $2,0\ \mu m$ gekennzeichnete Partikelgrößenverteilung aufweisen.

6. Verfahren zur Herstellung einer sterilen Mikrosphären-basierten Dermalfüllstoffformulierung nach Anspruch 4 oder 5, umfassend die Schritte:

(A) In-Kontakt-Bringen einer fettsäuregepfropften Hyaluronsäure mit einem Hyaluronsäure-Grundgerüst aus 1 250 bis 12 500 sich wiederholenden Disaccharid-Einheiten, wobei die Fettsäure eine C6-C22-Fettsäure ist, mit einer wässrigen Lösung, um eine Mikrosphären-basierte Formulierung von fettsäuregepfropfter Hyaluronsäure zu erhalten,

(B) gegebenenfalls Unterziehen der Mikrosphären-basierten Formulierung von fettsäuregepfropfter Hyaluronsäure einer Hochdruckhomogenisierungsbehandlung (high pressure homogenization; HPH),

(C) Sterilisieren der gegebenenfalls HPH-behandelten Mikrosphären-basierten Formulierung, um eine sterile Mikrosphären-basierte Dermalfüllstoffformulierung zu erhalten.

7. Sterile, vernetzte, Hyaluronsäure-basierte Dermalfüllstoffformulierung, umfassend Hyaluronsäure, die mit einem Vernetzungsmittel, insbesondere 1,4-Butandioldiglycidylether (BDDE), vernetzt ist, und eine fettsäuregepfropfte Hyaluronsäure mit einem Hyaluronsäure-Grundgerüst aus 1250 bis 12500 sich wiederholenden Disaccharid-Einheiten, wobei die Fettsäure eine C6-C22-Fettsäure ist.

8. Sterile, vernetzte, Hyaluronsäure-basierte Dermalfüllstoffformulierung nach Anspruch 7, wobei die vernetzte HA in einer Konzentration von 1 mg/ml bis 50 mg/ml vorliegt und/oder wobei die fettsäuregepfropfte Hyaluronsäure in einer Konzentration von 0,0001 Gew.-% bis 20 Gew.-% vorliegt.

9. Sterile Dermalfüllstoffformulierung nach einem der Ansprüche 4, 5, 7 und 8, die ferner ein Anästhetikum, insbesondere Lidocain, und/oder eine oder mehrere weitere Substanzen ausgewählt aus der Gruppe bestehend aus Polyolen, Vitaminen, Aminosäuren, Metallen, Antioxidantien, Hydroxyapatitpartikeln und Mineralsalzen, umfasst.

10. Verfahren zur Herstellung einer sterilen, vernetzten, Hyaluronsäure-basierten Dermalfüllstoffformulierung nach einem der Ansprüche 7 bis 9, umfassend die Schritte:

(i) Bereitstellen einer fettsäuregepfropften Hyaluronsäure mit einem Hyaluronsäure-Grundgerüst aus 1250 bis 12500 sich wiederholenden Disaccharid-Einheiten, wobei die Fettsäure eine C6-C22-Fettsäure ist,

(ii) Bereitstellen einer vernetzten Hyaluronsäure,

(iii) Kombinieren der fettsäuregepfropften Hyaluronsäure und der vernetzten Hyaluronsäure, um eine vernetzte, Hyaluronsäure-basierte Dermalfüllstoffformulierung zu erhalten, und

(iv) Sterilisieren der vernetzten, Hyaluronsäure-basierten Dermalfüllstoffformulierung, um eine sterile, vernetzte, Hyaluronsäure-basierte Dermalfüllstoffformulierung zu erhalten.

11. Verfahren nach einem der Ansprüche 1, 6 und 10, Verwendung nach Anspruch 2 oder 3 oder Formulierung nach einem der Ansprüche 4, 5, 7, 8 und 9, wobei die Fettsäure eine C8-C18-Fettsäure, vorzugsweise eine gesättigte oder ungesättigte, lineare C6-C22-Fettsäure, insbesondere eine gesättigte oder ungesättigte, lineare C8-C18-Fettsäure ist.

12. Verfahren nach einem der Ansprüche 1, 6 und 10, Verwendung nach Anspruch 2 oder 3 oder Formulierung nach einem der Ansprüche 4, 5, 7, 8 und 9, wobei der Modifikationsgrad der Hyaluronsäure mit Fettsäure 1 % bis 30 % beträgt.

13. Vorgefüllte Spritze umfassend eine sterile Dermalfüllstoffzusammensetzung nach einem der Ansprüche 4, 5, 7, 8 und 9.

14. Verwendung einer sterilen Dermalfüllstoffformulierung nach einem der Ansprüche 4, 5, 7, 8 und 9 für kosmetische Behandlungen, insbesondere zur Augmentation oder Auffüllung von Falten oder feinen Linien der Haut, zum Auffüllen von Hautvertiefungen, zum Abdecken von Narben, zur Vergrößerung des Lippenvolumens, zur Augmentation der Wangen, zur Nasenkorrektur und/oder zur Verbesserung der Hautfeuchtigkeit und der Hautbeschaffenheit.

**Revendications**

1. Procédé de préparation d'un acide hyaluronique greffé par un acide gras ayant un squelette d'acide hyaluronique de 1 250 à 12 500 unités disaccharidiques de répétition, dans lequel l'acide gras est un acide gras en $C_6$ à $C_{22}$, comprenant les étapes consistant à :

(a) disperser l'acide hyaluronique dans un solvant organique, en particulier un solvant non polaire, pour obtenir une dispersion organique,
(b) ajouter des chlorures d'acide gras à la dispersion organique,
(c) permettre aux chlorures d'acide gras de s'estérifier avec l'acide hyaluronique dans des conditions de réaction suffisantes pour former un acide hyaluronique greffé par un acide gras, et
(d) isoler l'acide hyaluronique greffé par un acide gras,

dans lequel l'acide hyaluronique dispersé dans l'étape (a) est prétraité par dispersion de l'acide hyaluronique natif, en particulier des fibres d'acide hyaluronique natif, dans une solution aqueuse, ce qui permet d'obtenir ainsi une dispersion aqueuse et de lyophiliser la dispersion aqueuse afin de produire un acide hyaluronique lyophilisé qui est ensuite dispersé dans l'étape (a).

2. Utilisation d'un acide hyaluronique greffé par un acide gras ayant un squelette d'acide hyaluronique de 1 250 à 12 500 unités disaccharidiques de répétition, dans laquelle l'acide gras est un acide gras en $C_6$ à $C_{22}$, dans la préparation d'une formulation de produit de comblement dermique.

3. Utilisation selon la revendication 2, dans laquelle ledit acide hyaluronique greffé par un acide gras est ajouté à un produit de comblement dermique à base de HA pour agir comme lubrifiant.

4. Formulation de produit de comblement dermique stérile à base de microsphères, comprenant des microsphères d'acide hyaluronique greffé par un acide gras, l'acide hyaluronique greffé par un acide gras ayant un squelette d'acide hyaluronique de 1 250 à 12 500 unités disaccharidiques de répétition, dans laquelle l'acide gras est un acide gras en $C_6$ à $C_{22}$.

5. Formulation de produit de comblement dermique stérile à base de microsphères selon la revendication 4, dans laquelle les microsphères d'acide hyaluronique greffé par un acide gras ont une distribution de taille de particules **caractérisée par** une valeur de D50 de 0,1 $\mu$m à 2,0 $\mu$m.

6. Procédé de préparation d'une formulation de produit de comblement dermique stérile à base de microsphères selon la revendication 4 ou 5, comprenant les étapes consistant à :

(A) mettre en contact un acide hyaluronique greffé par un acide gras ayant un squelette d'acide hyaluronique de 1 250 à 12 500 unités disaccharidiques de répétition, dans lequel l'acide gras est un acide gras en $C_6$ à $C_{22}$, avec une solution aqueuse afin d'obtenir une formulation à base de microsphères d'acide hyaluronique greffé par un acide gras,
(B) éventuellement soumettre la formulation à base de microsphères d'acide hyaluronique greffé par un acide gras à un traitement d'homogénéisation à haute pression (HPH), et
(C) stériliser la formulation à base de microsphères éventuellement traitée par HPH pour obtenir une formulation de produit de comblement dermique stérile à base de microsphères.

7. Formulation de produit de comblement dermique stérile à base d'acide hyaluronique réticulé, comprenant un acide hyaluronique réticulé avec un agent de réticulation, en particulier l'éther diglycidique du 1,4-butanediol (BDDE), et un acide hyaluronique greffé par un acide gras ayant un squelette d'acide hyaluronique de 1 250 à 12 500 unités disaccharidiques de répétition, dans laquelle l'acide gras est un acide gras en $C_6$ à $C_{22}$.

8. Formulation de produit de comblement dermique stérile à base d'acide hyaluronique réticulé selon la revendication 7, dans laquelle le HA réticulé est présent à une concentration de 1 mg/ml à 50 mg/ml et/ou dans laquelle l'acide hyaluronique greffé par un acide gras est présent à une concentration de 0,0001 % en poids à 20 % en poids.

9. Formulation de produit de comblement dermique stérile selon l'une quelconque des revendications 4, 5, 7 et 8, comprenant en outre un agent anesthésique, en particulier la lidocaïne, et/ou une ou plusieurs autres substances choisies parmi le groupe constitué de polyols, de vitamines, d'acides aminés, de métaux, d'antioxydants, de particules d'hydroxyapatite et de sels minéraux.

10. Procédé de préparation d'une formulation de produit de comblement dermique stérile à base d'acide hyaluronique réticulé selon l'une quelconque des revendications 7 à 9, comprenant les étapes consistant à :

(i) fournir un acide hyaluronique greffé par un acide gras ayant un squelette d'acide hyaluronique de 1 250 à 12 500 unités disaccharidiques de répétition, dans lequel l'acide gras est un acide gras en $C_6$ à $C_{22}$,

(ii) fournir un acide hyaluronique réticulé,

(iii) combiner l'acide hyaluronique greffé par un acide gras et l'acide hyaluronique réticulé pour obtenir une formulation de produit de comblement dermique à base d'acide hyaluronique réticulé, et

(iv) stériliser la formulation de produit de comblement dermique à base d'acide hyaluronique réticulé pour obtenir une formulation de produit de comblement dermique stérile à base d'acide hyaluronique réticulé.

11. Procédé selon l'une quelconque des revendications 1, 6 et 10, utilisation selon la revendication 2 ou 3, ou formulation selon l'une quelconque des revendications 4, 5, 7, 8 et 9, dans lequel l'acide gras est un acide gras en $C_8$ à $C_{18}$, préférentiellement un acide gras linéaire en $C_6$ à $C_{22}$ saturé ou insaturé, en particulier un acide gras linéaire en $C_8$ à $C_{18}$ saturé ou insaturé.

12. Procédé selon l'une quelconque des revendications 1, 6 et 10, utilisation selon la revendication 2 ou 3, ou formulation selon l'une quelconque des revendications 4, 5, 7, 8 et 9, dans lequel le degré de modification de l'acide hyaluronique avec l'acide gras est de 1 % à 30 %.

13. Seringue préremplie, comprenant une composition de produit de comblement dermique stérile selon l'une quelconque des revendications 4, 5, 7, 8 et 9.

14. Utilisation d'une formulation de produit de comblement dermique stérile selon l'une quelconque des revendications 4, 5, 7, 8 et 9 pour des traitements cosmétiques, en particulier pour le comblement des rides ou ridules de la peau, le comblement des affaissements cutanés, le masquage des cicatrices, l'augmentation du volume des lèvres, l'augmentation des joues, les corrections nasales, et/ou l'amélioration de l'hydratation et de la texture de la peau.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014082609 A
- US 2011224164 A
- US 20100316683 A1
- WO 2013185934 A1

**Non-patent literature cited in the description**

- **FARKAS et al.** *Plast. Reconstr. Surg. - Global Open*, 2013, vol. 1, e8
- **SMEJKALOVA et al.** *Carbohydrate Polymers*, 2012, vol. 87, 1460-1466
- **HUERTA-ANGELES et al.** *Carbohydrate Polymers*, 2014, vol. 111, 883-891
- **HOENNSCHEIDT et al.** *Int. J. Pharm.*, 2015, vol. 478, 416-425
- **HOENNSCHEIDT et al.** *Biochem. Eng.*, 2013, vol. 79, 259-266
- **CHRISTOPH HOENNSCHEIDT**. Entwicklung kolloiddisperser Wirkstoffformulierungen auf Basis von Biopolymeren. Cuvillier, 2016, 176
- **EDSMAN et al.** Gel Properties of Hyaluronic Acid Dermal Fillers. *Dermatol. Surg.*, 2012, vol. 38, 1170-1179
- **GUARISE et al.** SEC determination of cross-link efficiency in hyaluronan fillers. *Carbohydrate Polymers*, 2012, vol. 88, 428-434
- **KENNE et al.** Modification and crosslinking parameters in hyaluronic acid hydrogels - Definitions and analytical methods. *Carbohydrate Polymers*, 2013, vol. 91, 410-418